# EUROPEAN PATENT APPLICATION

(11) **EP 1 650 195 A1**
(43) Date of publication of application: **26.04.2006**
(21) Application number: 04771023.1
(22) Date of filing: 29.07.2004
(51) Int. Cl.: C07D 237/14, C07D 401/06, A61K 31/50, A61K 31/501, A61P 1/16, A61P 9/10, A61P 13/04, A61P 13/12, A61P 31/06, A61P 35/00, A61P 43/00

(54) **METHOD OF INHIBITING PRODUCTION OF OSTEOPONTIN**

(30) Priority: 30.07.2003 US 490950 P
(71) Applicant: Kowa Co., Ltd., Naka-ku, Nagoya-shi, Aichi 460-8625 (JP)
(72) Inventor: SAEKI, Yukihiko, 5941118 (JP); TABUNOKI, Yuichiro, 2030051 (JP); KOSHI, Tomoyuki, 3530006 (JP)
(74) Representative: Hartz, Nikolai
(86) International application number: PCT/JP2004/010810
(87) International publication number: WO 2005/012259

(57) **Abstract**

This invention relates to a method of inhibiting OPN production, which comprises administering an effective amount of a pyridazine derivative represented by the following formula (I) or a salt thereof: 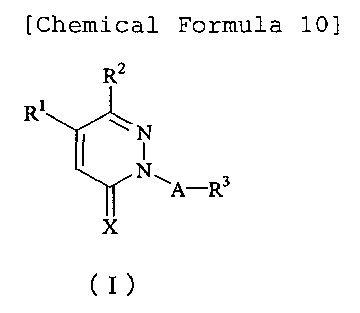 wherein:
R¹ means a substituted or unsubstitutedphenyl or pyridyl group;
R² means a substituted phenyl group;
R³ means a hydrogen atom or a substituted or unsubstituted phenyl or pyridyl group;
A means a single bond, a C₁₋₆ linear or branched alkylene group, or a C₂₋₉ linear or branched alkenylene group; and
X means an oxygen atom or a sulfur atom.

## Description

### Technical Field

This invention relates to a method of inhibiting the production of osteopontin, and specifically to a preventive and therapeutic method of diseases resulting from enhanced production of osteopontin, for example, multiple myeloma, urinary calculus and the like.

### Background Art

Ostepontin (hereinafter abbreviated as "OPN") is a secretory phosphoglycoprotein identified as an extracellular substrate of bone at first, and is expressed in cells such as osteoclasts, macrophages, activating T cells, smooth muscle cells and epicytes and also in tissues such as bones, the kidney, the placenta, smooth muscles and secretory epithelia. OPN has an arginin-glycin-aspartic acid (RGD) sequence, and in various cells, binds via αvβ1, β3 and β5 integrin to induce adhesion, chemotaxis and signal transduction. As effects of OPN, known physiological effects include promotion of bone resorption, promotion of vascularization, wound healing, and normal tissue repair processes in tissue destruction. Its connections to diseases have also been reported.

Known diseases to which increases in blood or tissue OPN are connected include post-PTCA restenosis (Non-patent Document 1), kidney disease (Non-patent Document 2), tuberculosis (Non-patent Document 3), sarcoidosis (Non-patent Document 4), chronic liver diseases such as cirrhosis (Non-patent Document 5), the following various cancers: colorectal cancer (Non-patent Document 6), ovarian cancer (Non-patent Document 7), prostatic cancer (Non-patent Document 8), breast cancer (Non-patent Document 9) and so on, urinary calculus (Non-patent Document 10) and the like, and myelomatous tumors (especially multiple myeloma) to be described subsequently in Examples. Realization of inhibition of OPN production or impediment to OPN function is expected to bring about preventive or therapeutic effects for these diseases.

Known OPN production suppressors or inhibitors include PPARγ agonists (Non-patent Document 11), HMG-CoA reductase inhibitors (Non-patent Document 12), etc. As PPARγ agonists, troglitazone, pioglitazone, rosiglitazone and the like can be mentioned. As HMG-CoA reductase inhibitors, rosvastatin, lovastatin, simvastatin, pravastatin, fulvastatin, atorvastatin, cerivastatin, pitavastatin, mevastatin and the like can bementioned. Except for such PPARγagonists andHMG-CoA reductase inhibitors, however, not many compounds are known to have OPN production inhibiting effect.
Non-patent Document 1: Circ. Res. **91**(1), 77-82, Jul. 12, 2002
Non-patent Document 2: Am. J. Hypertens., **16** (3), 214-22, Mar., 2003
Non-patent Document 3: Am. J. Respir. Crit. Care Med. , **167**(10), 1355-9, May 15, 2003
Non-patent Document 4: Lung, 179(5), 279-91, 2001
Non-patent Document 5: Biochem. Biophys. Res. Commun. **256**(3), 527-31, Mar. 24, 1999
Non-patent Document 6: J. Natl. Cancer Inst., **94**(7), 513-21, Apr. 3, 2002
Non-patent Document 7: JAMA, **287**(13), 1671-9, Apr. 3, 2002
Non-patent Document 8: Clin. Cancer Res., **5**(8),**22**71-7, Aug. 1999
Non-patent Document 9: Clin. Cancer Res., **3** (4), 605-11, Apr. 1997
Non-patent Document 10: J. Biol. Chem., **268**(20), 15180-4, Jul. 15, 1993
Non-patent Document 11: Circ. Res.,**90**, 348-355, 2002 Non-patent Document 12: Br. J. Pharmacol., **133**, 83-88,2001

### Disclosure of the Invention

An object of this invention is to provide a novel method for the inhibition of OPN production.

With the foregoing circumstances in view, the present inventors have conducted an extensive investigation. As a result, it has been unexpectedly found that the below-described compounds of the formula (I) known to have interleukin-1β inhibiting effect are also equipped with OPN production inhibiting effect, leading to the completion of the present invention.

Described specifically, the present invention provides a method of inhibiting OPN production, comprising administering an effective amount of a pyridazine derivative represented by the following formula (I) or a derivative thereof:

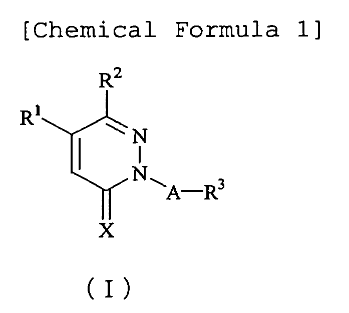

wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

The present invention also provides an OPN production inhibitor and a preventive and therapeutic agent for a disease resulting from enhanced OPN production, both of which comprise as an active ingredient a pyridazine derivative represented by the formula (I) or a salt thereof.

The present invention also provides use of a pyridazine derivative represented by the formula (I) or a salt thereof for the production of an OPN production inhibitor and a preventive and therapeutic agent for a disease resulting from enhanced OPN production.

The present invention further provides an OPN production inhibitor composition and a preventive and therapeutic agent composition for a disease resulting from enhanced OPN production, both of which comprise a pyridazine derivative represented by the formula (I) or a salt thereof and a pharmaceutically acceptable carrier.

The present invention still further provides a therapeutic method of a disease resulting from enhanced OPN production, which comprises administering a pyridazine derivative represented by the following formula (I) or a derivative thereof.

According to the present invention, it is possible to provide an osteopontin production inhibitor useful for the prevention and treatment of diseases associated with the production of osteopontin, for example, multiple myeloma and urinary calculus.

### Brief Description of the Drawings

[FIG.1] FIG. 1 shows the results of immunocytochemical staining of osteopontin in bone marrow cells derived from multiple myeloma (left) and a control group (MGUS) (right) .
[FIG. 2] FIG. 2 shows the results of immunocytochemical staining of osteopontin in MGUS (A), myelodysplastic syndrome (MDS) (B), idiopathic thrombocytopenic purpura (ITP) (C), acute myelocytic leukemia (AML) (D), and hereditary spherocytosis (HSC) (E).
[FIG. 3] FIG. 3 shows the expression of osteopontin (OPN) and GAPDH by RT-PCR in various cell lines.
[FIG. 4] FIG. 4 shows the expression of osteopontin (OPN) by Western blotting in various cells.
[FIG. 5] FIG. 5 diagrammatically shows the distributions of plasma osteopontin concentrations in a multiple myeloma patient (MM), MGUS and healthy subject.
[FIG. 6] FIG. 6 diagrammatically shows the plasma osteopontin concentrations of a multiple myeloma patient in Stage I, Stage II (inactivity) and Stage III (activity).
[FIG. 7] FIG. 7 diagrammatically shows differences in plasma osteopontin concentration depending on the existence or non-existence of bone pain on multiple myeloma patients.
[FIG. 8] FIG. 8 diagrammatically shows differences in plasma osteopontin concentration depending on the existence or non-existence of an osteoclastic bone resorption pattern on multiple myeloma patients.

### Best Modes for Carrying out the Invention

As disclosed in WO 99/25697, the pyridazine derivatives represented by the formula (I) or their salts, which are useful in the present invention, are known to have excellent interleukin-1β production inhibiting effect, and are useful as preventive and therapeutic agents for various diseases such as immune diseases and inflammatory diseases caused by enhanced interleukin-1β production. However, absolutely nothing is known as to whether or not the compounds represented by the formula (I) have OPN production inhibiting effect. It is to be noted that the details of the description in WO 99/25697, such as the preparation process of the compounds (I) and the formulationmethod of preparations with the compounds (I) contained as active ingredients, are incorporated herein by reference.

In the formula (I), R¹ is a phenyl or pyridyl group which may be substituted by 1 to 3 substituents selected fromhalogen atoms and C₁₋₆ alkoxy groups. As the halogen atoms, fluorine atoms, chlorine atoms, bromine atoms, iodine atoms and the like can be mentioned. Examples of the C₁₋₆ alkoxy groups include methoxy groups, ethoxy groups, propoxy groups, isopropoxy groups, and the like. Preferably, these substituents may each exists at the 3-, 4- or 5-position.

R² is a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups. As the C₁₋₆ alkoxythio group(s) as substituent(s) on the phenyl group of R², methylthio group(s), ethylthio group(s), propylthio group(s), isopropylthio group (s) and/or the like canbementioned. As the halogen atom(s) and/or C₁₋₆ alkoxy group(s) as substituent (s) on the phenyl group of R², on the other hand, similar atoms and groups as mentioned above with respect to R¹ can be mentioned. These substituent(s) may preferably exist at the 4-position only, at the 3-position and 4-position, or at the 3-position, 4-position and 5-position.

R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group.

As the C₁₋₆ alkoxy groups and halogen atoms, similar groups and atoms as mentioned above with respect to R¹ can be mentioned. As the C₁₋₆ alkylthio groups, similar alkylthio groups as mentioned above with respect to R² can be mentioned. As the halogenated C₁₋₆ alkyl groups, C₁₋₆ alkyl groups with 1 to 3 halogen atoms represented by R¹ and substituted thereon can be mentioned. As the C₃₋₆ cycloalkyl group, a cyclopropyl group, cyclobutyl group, cyclopentyl group or cyclohexyl group can be mentioned.

Examples of the C₁₋₆ alkyl group (s) include methyl group (s), ethyl group (s), n-propyl group (s), isopropyl group(s), and n-butyl group(s). Examples of the C₂₋₇ alkoxycarbonyl group(s) include methoxycarbonyl group(s), ethoxycarbonyl group(s), and propoxycarbonyl group(s) . The C₁₋₆ alkylamino group (s) each contains one or two C₁₋₆ alkyl groups, and examples include methylamino group(s), dimethylamino group(s), ethylamino group(s), and propylamino group(s).

As group (s) which can substitute on the piperidino, piperidyl, piperadino or morpholino group, halogen atom (s), C₁₋₆ alkoxy group (s) and/or C₁₋₆ alkyl group (s) can be mentioned. As group (s) which can substitute on the aminocarbonyl group, C₆₋₁₂ aralkyl group (s) such as benzyl group(s) and/or phenethyl group(s) can be mentioned in addition to C₁₋₆ alkyl group (s) and/or C₁₋₆ alkoxy group(s). As the C₂₋₇ alkylcarbonyl group, a methylcarbonyl group, ethylcarbonyl group or the like can be mentioned.

Among those indicated byA, the linear or branched C₁₋₆ alkylene group can be a methylene group, ethylene group, trimethylene group, or the like. As the linear or branched C₂₋₉ alkenylene group, one having 2 to 9 carbon atoms and 1 to 3 double bonds is preferred. Examples include an ethenylene group, propenylene group, butenylene group, and butadienylene group.

More preferred are those of the formula (I) in which R¹ is a phenyl or pyridyl group substituted at the 4-position thereof with a halogen atom selected from fluorine chlorine or bromine or a C₁₋₆ alkoxy group; R² is a phenyl group substituted at the 4-position thereof with a C₁₋₆ alkoxy group or a C₁₋₆ alkylthio group; R³ is a hydrogen atom or a phenyl or pyridyl group substituted by one to three halogen atoms; and A is a C₁₋₂ alkylene group or C₃₋₄ alkenylene group.

Most preferred are those of the formula (I) in which R¹ is a phenyl or pyridyl group substituted at the 4-position thereof with a chlorine atom or a methoxy group; R² is a phenyl group substituted at the 4-position thereof with a methoxy group or methylthio group; R³ is a hydrogen atom, phenyl group, 4-chlorophenyl group, 2-pyridyl group, or 3-pyridyl group; andA is a methylene group, ethylene group or 2-propenylene group.

More specifically, particularly preferred is the method of claim 1 in which the active ingredient is 5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

No particular limitation is imposed on the salt of the pyridazine derivative (I), said salt being useful in the present invention, insofar as the salt is pharmaceutically acceptable. Examples include acid addition salts of mineral acids, such as the hydrochloride, hydrobromide, hydroiodide, sulfate, nitrate and phosphate; and acid addition salts of organic acids, such as the benzoate, methanesulfonate, ethanesulfonate, benzenesulfonate, p-toluenesulfonate, oxalate, maleate, fumarate, tartrate and citrate.

The compounds useful in the present invention can also exist in the form of solvates represented by hydrates or in the form of keto-enol tautomers, and such solvates and tautomers shall also be encompassed by the present invention.

The pyridazine derivatives (I) and their salts have excellent OPN production inhibiting effect as will be demonstrated subsequently in Examples, and are useful as preventive and therapeutic agents for diseases resulting from enhanced OPN production, for example, post-PTCA restenosis, kidney disease, tuberculosis, sarcoidosis, chronic liver diseases such as cirrhosis, the following various cancers: colorectal cancer, ovarian cancer, prostatic cancer, breast cancer and so on, urinary calculus and the like, and myelomatous tumors (especially, multiple myeloma).

The drug according to the present invention contains the pyridazine derivative (I) or its salt as an active ingredient. Examples of its administration route include oral administration by tablets, capsules, granules, a powder, a syrup or the like and parenteral administration by an intra-vascular injection, a muscular injection, suppositories, an inhalant, a transdermal system, an eye drop, a nose drop or the like. Upon formulation of the drug composition into such various preparation forms, pharmaceutically acceptable carriers can be added to the active ingredient. As such carriers, excipients, binders, extenders, disintegrants, surfactants, lubricants, dispersants, buffering agents, preservatives, corrigents, fragrances, coating agents, carriers, diluents and the like can be used in combination as need.

The dose of the drug according to the present invention differs depending on the age, weight, conditions, administration form, administration frequency and the like. In general, however, it is preferred to orally administer or parenterally administer the drug at once or in several portions to an adult at a dose of 0.01 to 1,000 mg, preferably, 0.1 to 100 mg in terms of the pyridazine derivative (I) or its salt.

### Examples

The present invention will hereinafter be described in detail based on Examples. It should, however, be borne in mind that the present invention is not limited to or by the following Examples.

Synthesis Example 1
Synthesis of
5-(4-chlorophenyl)-6-[4-(methylthio)phenyl]-2-(2-py ridylmethyl)-2H-pyridazine-3-thione methanesulfonate
Synthesis of
5-(4-chlorophenyl)-6-[4-(methylthio)phenyl]-2-(2-pyridylmethyl)-2H-pyridazin-3-one
Potassium carbonate (525 mg, 3.78 mmol) and 2-picolylchloride hydrochloride (300 mg, 1.83 mmol) were added to a solution of 5-(4-chlorophenyl)-6-[4-(methylthio)phenyl]-2H-pyri dazin-3-one (500 mg, 1.52 mmol) in N,N-dimethylformamide (10 mL), followed by stirring at 80°C for 12 hours. Chloroform (50 mL) was added to the reaction mixture. The mixture was washed successively with water and saturated brine, and was dried over anhydrous sodium sulfate. The solvent was distilled off under reduced pressure. The residue was isolated and purified by column chromatography on silica gel (ethyl acetate/hexane = 1/1 to 2/1) to afford the title compound as a slightly-yellow amorphous (623 mg, 97.5%).

¹H-NMR (CDCl₃) δ: 2.45(3H, s), 5.58(2H, s), 6.96(1H, s), 7.06-7.11(6H, m), 7.21(1H, m), 7.27-7.33(3H, m), 7.67(1H, m), 8.59(1H, m).
IR(KBr) cm⁻¹: 1667, 1593, 1584, 1492, 1092.
Mass(m/z): 421(M⁺), 419(M⁺).

· Synthesis of
5-(4-chlorophenyl)-6-[4-(methylthio)phenyl]-2-(2-pyridylmethyl)-2H-pyridazine-3-thione
Lawesson's reagent (400 mg, 0.989 mmol) was added
to a solution of 5-(4-chlorophenyl)-6-[4-(methylthio)phenyl]-2-(2-py ridylmethyl)-2H-pyridazin-3-one (345 mg, 0.822 mmol) in toluene (5 mL), followed by stirring at 100°C for 2 hours. Chloroform (30 mL) was added to the reaction mixture. The mixture was washed successively with water and saturated brine, and was dried over anhydrous sodium sulfate. The solvent was distilled off under reduced pressure. The residue was isolated and purified by column chromatography on silica gel (chloroform/hexane = 4/1 to chloroform) to afford the title compound as a yellow amorphous (331 mg, 92.4%).

¹H-NMR (CDCl₃) δ: 2.46(3H, s), 6.09(2H, s), 7.09-7.14(4 H, m), 7.21(1H, m), 7.26-7.34(5H, m), 7.67(1H, m), 7.82(1H, s), 8.60(1H, m).
IR(KBr) cm⁻¹: 1593, 1473, 1159, 1099.
Mass (m/z) : 437(M⁺), 435(M⁺).

. Synthesis of
5-(4-chlorophenyl)-6-[4-(methylthio)phenyl]-2-(2-pyridylmethyl)-2H-pyridazin-3-thione methanesulfonate
Al mmol/mLmethanesulfonic acid-dioxane solution (0.53 mL, 0.53 mmol) was added to an ice-cold solution of 5-(4-chlorophenyl)-6-[4-(methylthio)phenyl]-2-(2-py ridylmethyl)-2H-pyridazine-3-thione (210 mg, 0.582 mmol) in methanol (2 mL), followed by stirring at the same temperature for 5 minutes. The solvent was distilled off under reduced pressure, and the residue was crystallized frommethanol-ether to afford the title compound as a yellow crystalline powder (248 mg, 96.8%).

Melting point: 215.0-217.4°C
¹H-NMR (DMSO-d₆) δ: 2.37(3H, s), 2.45(3H, s), 6.08(2H, s),7.15(2H, d, J = 8.8 Hz), 7.19(2H, d, J = 8.8 Hz), 7.31 (2H, d, J = 8.8 Hz), 7.45 (2H, d, J = 8.8 Hz), 7. 53-7.58(2H, m), 7.80(1H, s), 8.06(1H, m), 8.68(1H, m).
IR(KBr) cm⁻¹: 1228, 1169, 1100.

Synthesis Example 2
Synthesis of
5-(4-chlorophenyl)-6-[4-(methylthio)phenyl]-2-(3-py ridylmethyl)-2H-pyridazin-3-one methanesulfonate
Following the procedure of Example 1-3) and conducting crystallization from methanol-ether, the title compound was afforded as a slightly-brown crystalline powder (268 mg, 96.5%) from 5-(4-chlorophenyl)-6-[4-(methylthio)phenyl]-2-(3-py ridylmethyl)-2H-pyridazin-3-one (226 mg, 0.583 mmol) and 1 mmol/mL methanesulfonic acid-dioxane solution (0.59 mL, 0.59 mmol).

Melting point: 184.4-187.1°C
¹H-NMR (DMSO-d₆) δ: 2.35(3H, s), 2.45(3H, s), 5.53 (2H, s), 7.06(1H, s), 7.10(2H, d, J = 8.6 Hz), 7.17(2H, d, J = 8.6 Hz), 7.25(2H, d, J = 8.6 Hz), 7.44(2H, d, J = 8.6 Hz), 7.93(1H, dd, J = 5.6, 8.1 Hz), 8.42(1H, m), 8.66(1H, dd, J = 1.2, 5.6 Hz), 8.94(1H, d, J = 2. 0 Hz).
IR(KBr) cm⁻¹: 1665, 1227, 1212, 1194, 1156.

Example 1 (Immunocytochemistry for osteopontin)
The expressions of osteopontin in bone marrow cells collected from three typical multiple myeloma patients were studied by an immunocytochemical procedure making use of the avidin-biotin-peroxidase method. As the control, bone marrow cells from five patients with different hematologic diseases including monoclonal gammopaties with uncertain significance (MGUS: an increase in a monoclonal immunoglobulin is observed, but not to such an extent as meeting a diagnostic standard for multiple myeloma)were usued. Bone marrow cells were isolated by density-gradient centrifugation. Bone marrow cells (1 × 10⁵) derived from each patient and prepared from the isolated bone marrow cells were fixed with Cytospin 2 (Shandon Soutern Products Ltd., Cheshire, UK) on a glass slide. The slide was stored at -80°C until use. A mouse anti-human osteopontin monoclonal IgG antibody (4C1) prepared by Kon, et al. (J. CellularBiochemistry, **84**, 420-432, 2002) was used as a primary antibody. A mouse IgG antibody (Pharmingen, San Diego, USA), which was unrelated to osteopontin and was of the same concentration), was employed as a primary antibody (negative control). A biotinylated horse anti-mouse IgG antibody (Vector, Laboratories, Burlingame, USA) was used as a secondary antibody. The Cytospin slides were fixed with cold isopropanol for 2 minutes. After subjected to blocking with 10% normal horse serum, the cells were reacted with 4C1 or the negative control antibody at 4°C overnight. Endogenous peroxidase activity was blocked by applying 0.3% hydrogen peroxidase dissolved in methanol for 30 minutes. Subsequent to washing with PBS (phosphated buffer), the biotinylated secondary antibody was reacted at room temperature for 2 hours. After washing, an avidin-horseradish peroxidase complex (VECTASTAIN Elite ABC kit, Vector Laboratories, Burlingame, USA) was reacted for 1 hour. Staining was then effected with a substrate making use of diaminobenzene tetrahydrochloride (DBA), and the Giemsa staining was then conducted to count the cells.

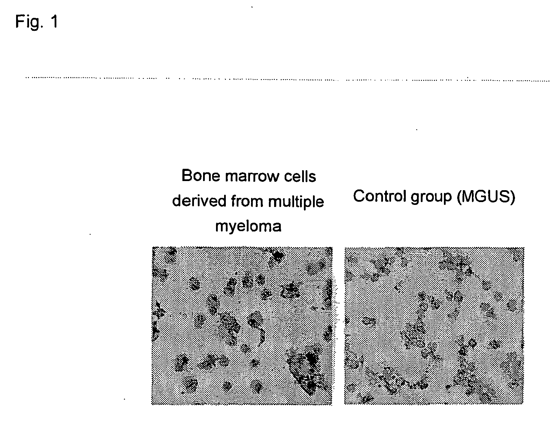

As a result, as shown in FIG. 1, most of bone marrow cells which had the form of typical myeloma cells were stained brown with 4C1, the mouse anti-human osteopontin monoclonal IgGantibody (leftmicrophoto). On the other hand, no staining was observed with the control antibody (right microphoto).

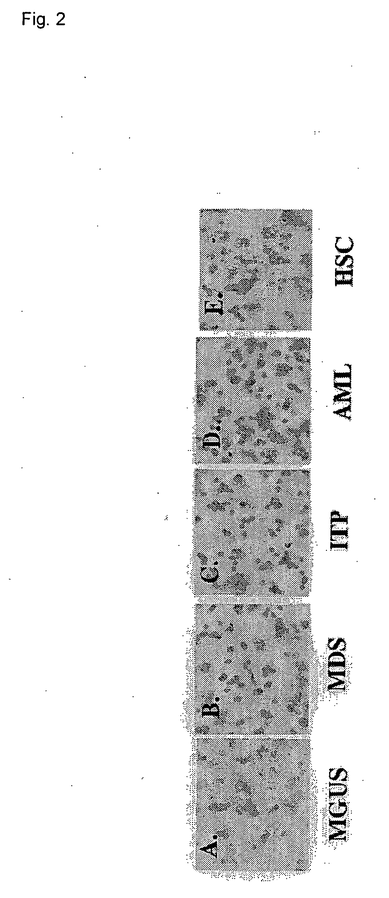

As shown in FIG. 2, any stain that indicates the expression of osteopontin was not observed on the bone marrow cells of any one of the sources, that is, MGUS (microphoto A), myelodysplastic syndrome (microphoto B), idiopathic thrombocytopenic purpura (microphoto C), acute myelocytic leukemia (microphoto D), and hereditary spherocytosis (microphoto E).

It has, therefore, been ascertained that osteopontin is expressed specifically in myeloma cells.

Example 2 (Analysis of osteopontin by RT-PCR)
The expressions of mRNA of osteopontin in cells of the B cell line in different stages (RPMI8226: myeloma cell line, Daudi: B lymphoblast cell line derived from Burkitt' s lymphoma, Ramos: B lymphoblast cell line derived from Burkitt's lymphoma, Raji: B lymphoblast cell line derived from Burkitt's lymphoma, Kopn-8:pre-B cell line, NALM-16: pro-B cell line, Reh: pro-B cell line) were studied by RT-PCR with specific primers designed from human osteopontin (sense primer: 5'-GGACTCCATT GACTCGAACG-3' (SEQ. NO. 1), antisense primer: 5'-TAATCTGGACTGCTTGTGGC-3' (SEQ. NO. 2)). From the respective cell lines, mRNAs (100 ng) were purified with TRIZOL reagent (Life Technologies, Rockville, USA). From them, individual cDNAs were synthesized. Using the osteopontin-specific primers, PCR was performed under conditions to be mentioned hereinafter. Specifically, denaturation was conducted at 94°C for 1 minute, followed by annealing at 57°C for 1 minute and further by extension at 72°C for 2 minutes. This cycle was repeated 30 times. As controls, primers specific to GAPDH (glyceraldehyde-3-phosphate dehydrogenase)(sense primer: 5'-AATTACCACAACCCCTACAAAC-3' (SEQ. NO. 3), antisense primer:5'-CAACTCTGCAACATCTTCCTC-3' (SEQ. NO. 4)) were used. The PCR products were subjected to electrophoresis in 2% agarose gel to confirm the existence or non-existence of any bands.

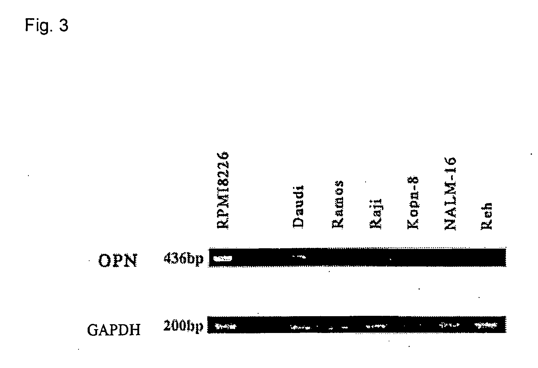

As a result, as shown in FIG. 3, distinct bands were observed on RPMI8226, a myeloma cell line, and some bands were also observed on Daudi. However, no osteopont in band was observed on the remaining cell lines which were not myeloma cell lines.

Example 3 (Analysis of osteopontin by Western blotting)
To study spontaneous production of osteopontin, a Western blotting analysis was performed using similar B cell lines of different stages as in Example 2. Cells of each B cell line was cultured *in vitro* for 3 days, and a culture supernatant was collected. After proteins were separated from the culture supernatant (20 µL) of the cells of each B cell line by 4-hour SDS-PAGE with a 4-20% density gradient gel of acrylamide, the proteins were transferred overnight at 4°C onto Immobilon P Membrane (Millipore, Bedford, USA) . The membrane with the proteins transferred thereon was subjected to blocking with a phosphated buffer (PBS) which contained 10% skim milk and 0.1% Tween 20. After washing the membrane, a rabbit anti-human osteopontin antibody (OPN2) prepared by Kon, et al. (J. Cell. Biochem., **77**, 487-498, 2000) was added and reacted overnight at 4°C. Subsequent to washing, an HRP-labeled goat anti-rabbit IgG antibody was added and reacted at room temperature for 1 hour. After washing, the film was developed overnight with Renaissance reagent (NEN Life Science Products, Boston, USA) to detect signals.

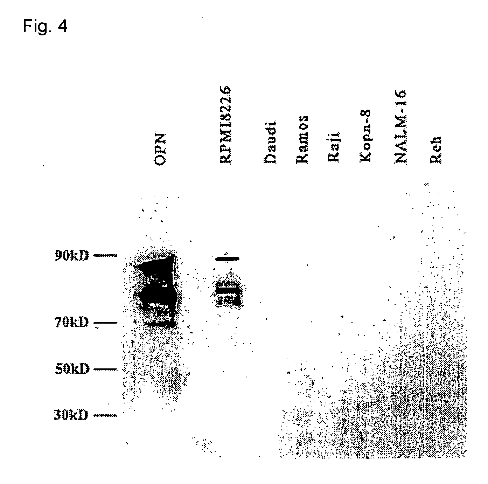

As a result, as shown in FIG. 4, the band of osteopontin was observed only on RPMI8226, and was not observed on the other cells.
From these FIG. 3 and FIG. 4, it is appreciated that osteopontin is expressed specifically in the myeloma cell line and is not expressed in other oncocyte lines.

Example 4 (Quantitation of plasma osteopontin by ELISA)
The plasma osteopontin concentrations of thirty multiple myeloma patients were measured using a human osteopontin ELISA kit (Immuno-Biological Laboratories, Gunma, Japan). As controls, plasmas collected from twenty-one MGUS patients and thirty healthy volunteers were used. Data were expressed in terms of "means ± standard error". Using the Mann-Whitney U Test, a test was conducted. Ap value smaller than 0.05 (a risk factor lower than 5%) was taken as having a significant difference.

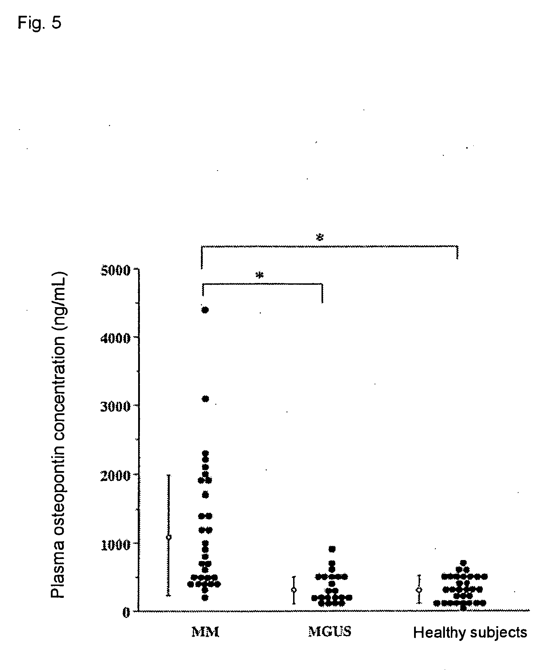

As a result, as shown in FIG. 5, the plasma osteopontin concentrations of multiple myeloma patients were found to have significantly higher values than those of the MGUS patients and healthy volunteers.
Plasma osteopontin concentration, means ± standard error, ng/mL: multiple myeloma: 1053 ± 957, MGUS: 355 ± 205, healthy volunteers: 309 ± 184.
Multiple myeloma vs MGUS and healthy volunteers: *p<0.05

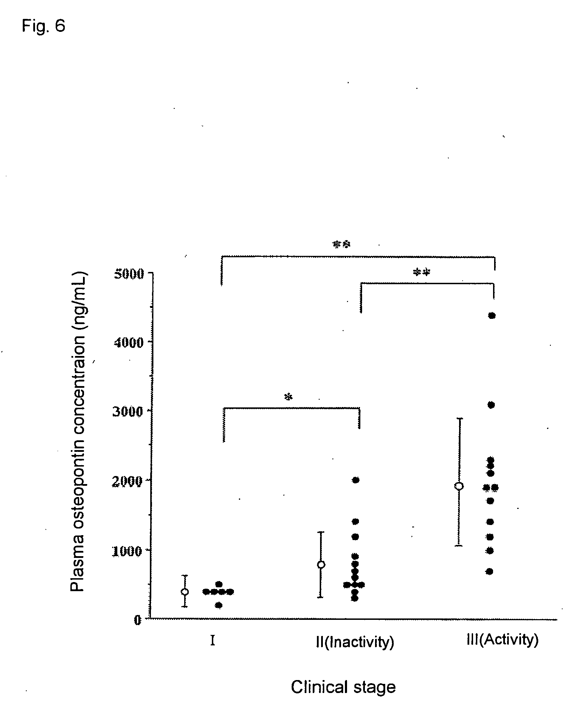

FIG. 6 diagrammatically shows the results of a comparison in plasma osteopontin concentration conducted by classifying multiple myeloma patients into three clinical stages of Stage I (6 patients), Stage II (inactivity) (12 patients) and Stage III (activity) (12 patients), according to classification of Durie & Salmon (Cancer, 36, 842-54, 1975) . As evident fromFIG. 6, the plasma osteopontin concentrations of the multiple myeloma patients showed significant, stage- or activity-dependent increases.
Plasma osteopontin concentration, means ± standard error, ng/mL: Stage I: 389 ± 89, Stage II (inactivity): 816 ± 446, Stage III (activity) : 1991 ± 953.
Stage II (inactivity) vs Stage I: *p<0.05
Stage III(activity) vs Stage I, Stage II (inactivity): *p<0.05

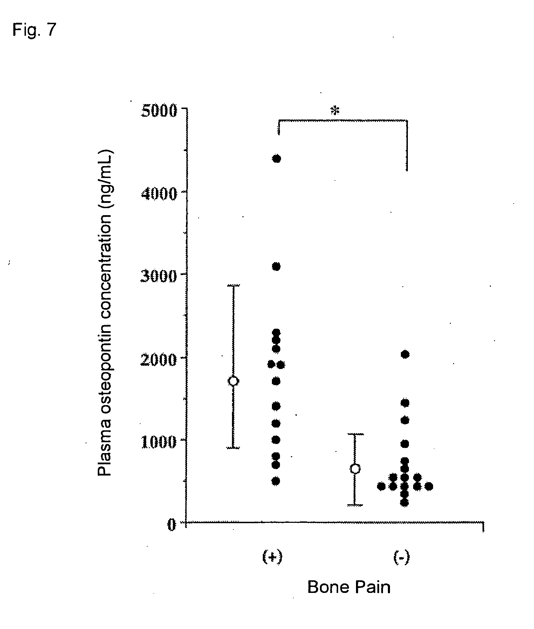

FIG. 7 diagrammatically shows the results of a comparison in the plasma osteopontin concentrations of multiple myeloma patients between two groups. One of the groups consisted of patients who felt substantially no bone pain, and the other group consisted of patients having marked bone pain. The plasma osteopontin concentrations of the patients suffering from marked bone pain were found to have significantly high values in comparison with the group who felt substantially no pain.
Plasma osteopontin concentration, means ± standard error, ng/mL: bone pain[-]: 776 ± 660, bone pain[+]: 1822 ± 994.
Bone pain[+] vs bone pain[-]: *p<0.05

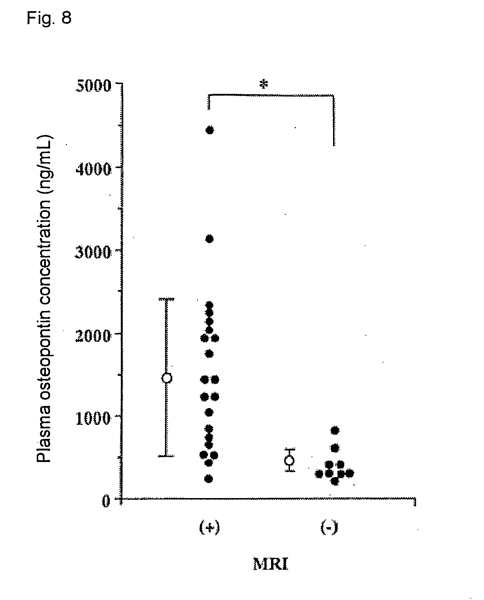

FIG. 8 diagrammatically shows the results of a comparison in the plasma osteopontin concentrations of multiple myeloma patients between two groups. One of the groups consisted of patients on whom substantially no osteoclastic bone resorption patterns were observed by magnetic resonance imaging (MRI) . The other group consisted of patients on whom marked osteoclastic bone resorption patterns were observed. The plasma osteopontin concentrations of the patients on whom marked osteoclastic bone resorption patterns were observed were found to have significantly high values in comparison with the group on whom substantially no osteoclastic bone resorption patterns were observed.
Plasma osteopontin concentration, means ± standard error, ng/mL: osteoclastic bone resorption pattern[-]: 486 ± 169, osteoclastic bone resorption pattern[+]: 1498 ± 486.
Osteoclastic bone resorption pattern[+] vs osteoclastic bone resorption pattern[-]: *p<0.05

Example 5 (Study on OPN production inhibiting effect by the use of cultured cells)
1) Used compounds
For the following study, the following individual compounds disclosed in International Publication No. WO 99/25697, specifically, the four compounds of Example 12
(5,6-bis(4-methoxyphenyl)-2-(4-chlorocinnamyl)-2H-p yridazin-3-one; hereafter referred to as "Compound 3"),
Example 51
(2-benzyl-5-(4-chlorophenyl)-6-[4-(methylthio)pheny 1]-2H-pyridazin-3-one; hereinafter referred to as "Compound 4"), Example 78 (2-(4-chlorobenzyl-6-(4-(methoxyphenyl)-5-(4-pyridy 1)-2H-pyridazin-3-one; hereinafter referred to as "Compound 5") and Example 163
(5,6-bis(4-methoxyphenyl)-2-ethyl-2H-pyridazin-3-on e; hereinafter referred to as "Compound 6") were synthesized by the disclosed processes and provided in addition to the compounds obtained above in Synthesis Examples 1 and 2 (hereinafter referred to as "Compound 1" and "Compound 2", respectively).

2) Preparation of solutions of the compounds
Compounds 1 to 6 were separately dissolved in dimethylsulfoxide (DMSO) to prepare 20 mmol/L solutions of the respective compounds. Aliquots of those solutions were diluted further to prepare 6, 2, 0. 6 and 0.2 mmol/L solutions of the respective compounds. The DMSO solutions of the respective compounds (1 to 6) (concentrations: 0.2 to 20 mmol/L) and DMSO (as a compound non-addition group) were separately diluted 1,000-fold with a culture medium (10% fetal-bovine-serum(FBS)-added RPMI-1640 medium) to prepare 0 (non-addition group), 0.2, 0.6, 2, 6 and 20 µmol/L solutions of the respective compounds (1-6).

3) Study on the OPN production inhibiting effect of the individual compounds in RPMI8226 cells
A 2 × 10⁵ cells/mL cell suspension of RPMI8226 cells was prepared, and was seeded 2 mL by 2 mL per well on a 6-well plates. To the wells, the individual solutions prepared in the procedure 2) (the solutions of Compounds 1 to 6, having concentrations of 0 (non-addition group), 0.2, 0.6, 2, 6, and 20 µmol/L, respectively) were added 2 mL by 2 mL per well, followed by mixing to bring the final concentrations of Compound 1-6 in the individual wells to 0 (non-added group), 0.1, 0.3, 1, 3 and 10 µmol/L. The cells of those conditions were cultured as prophase culture at 37°C for 3 days in the presence of 5% CO₂.

After the above-described culture, the cells in the respective wells were collected, and were counted well by well. Those cells were again suspended separately in aliquots of a 2 × 10⁵ cells/mL suspension, and the cells of the respective conditions were each seeded 0.5 mL by 0.5 mL per well in two wells. To the wells of the respective conditions, individual solutions prepared at the same concentrations as those at the time of the culture in a similar manner as in the prophase culture (individual solutions of Compounds 1 to 6, having concentrations of 0.2, 0.6, 2, 6 and 20 µmol/L, respectively) were added 0.5 mL by 0.5 mL per sell to the individual wells. After mixing, the cells were cultured as anaphase culture at 37°C for 3 days in the presence of 5% CO₂.

Subsequent to the completion of the anaphase culture, culture supernatants were separately collected from the respective wells, and the OPN concentrations in the individual culture supernatants were quantitated with an absorptiometer by ELISA (Human Osteopontin Measurement Kit IBL; IBL Co., Ltd.).

Using the SAS Preclinical Package, Version 5.0, the measurement values were analyzed by the following procedure. Described specifically, the concentrations were logarithmically converted (the compound non-addition group (0 µmol/L) was replaced by 1 pmol/L), and using the measurement values (6 concentrations, 2 measurement values per concentration, 12 measurement values in total), plotting into a logistic curve was conducted. From the curve, concentrations (IC₅₀) which give 50% reactivity were calculated. The results are presented in Table 1.

**[Table 1]**

| Compound (No.) | IC₅₀ (µmol/L) |
|---|---|
| 1 | 2.09 |
| 2 | 2.91 |
| 3 | 2.76 |
| 4 | 2.56 |
| 5 | 2.42 |
| 6 | 1.12 |

As presented in Table 1, Compounds 1 to 6 all showed excellent OPN production inhibiting effect.

## Claims

1. A method of inhibiting OPN production, comprising administering an effective amount of a pyridazine derivative represented by the following formula (I) or a derivative thereof: 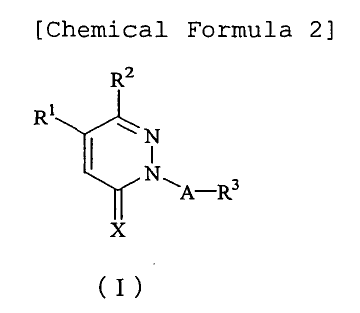 wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

2. The method of claim 1, wherein in the formula (I),
R¹ is a phenyl or pyridyl group which may be substituted at the 4-position thereof with a halogen atom selected from fluorine, chlorine or bromine or a C₁₋₆ alkoxy group;
R² is a phenyl group substituted at the 4-position thereof with a C₁₋₆ alkoxy group or a C₁₋₆ alkylthio group;
R³ is a hydrogen atom or a phenyl or pyridyl group which may be substituted by halogen atom or atoms; and
A is a C₁₋₃ alkylene group or C₃₋₄ alkenylene group.

3. The method of claim 1, wherein in the formula (I),
R¹ is a phenyl or pyridyl group which may be substituted at the 4-position thereof with a chlorine atom or a methoxy group;
R² is a phenyl group substituted at the 4-position thereof with a methoxy group or a methylthio group;
R³ is a hydrogen atom, phenyl group, 4-chlorophenyl group, 2-pyridyl group or 3-pyridyl group; and
A is a methylene group, ethylene group or 2-propenylene group.

4. The method of claim 1, wherein the active ingredient is
5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

5. An OPN production inhibitor, comprising as an active ingredient a pyridazine derivative represented by the following formula (I) or a derivative thereof: 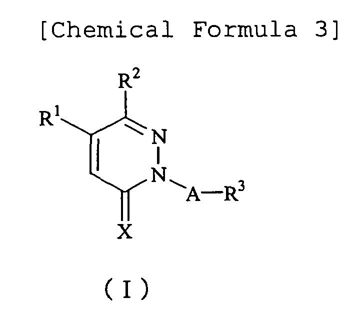 wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

6. The inhibitor of claim 5, wherein in the formula (I),
R¹ is a phenyl or pyridyl group which may be substituted at the 4-position thereof with a halogen atom selected from fluorine, chlorine or bromine or a C₁₋₆ alkoxy group;
R² is a phenyl group substituted at the 4-position thereof with a C₁₋₆ alkoxy group or a C₁₋₆ alkylthio group;
R³ is a hydrogen atom or a phenyl or pyridyl group which may be substituted by halogen atom or atoms; and
A is a C₁₋₃ alkylene group or C₃₋₄ alkenylene group.

7. The inhibitor of claim 5, wherein in the formula (I),
R¹ is a phenyl or pyridyl group which may be substituted at the 4-position thereof with a chlorine atom or a methoxy group;
R² is a phenyl group substituted at the 4-position thereof with a methoxy group or a methylthio group;
R³ is a hydrogen atom, phenyl group, 4-chlorophenyl group, 2-pyridyl group or 3-pyridyl group; and
A is a methylene group, ethylene group or 2-propenylene group.

8. The inhibitor of claim 5, wherein said active ingredient is
5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

9. A preventive and therapeutic agent for a disease resulting from enhanced OPN production, comprising as an active ingredient a pyridazine derivative represented by the following formula (I) or a derivative thereof: 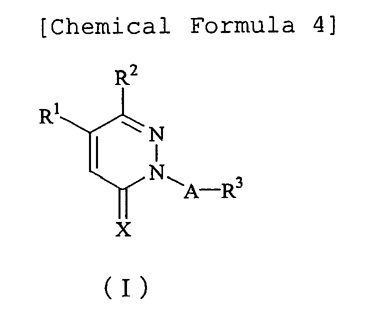 wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

10. The preventive and therapeutic agent of claim 9, wherein in the formula (I),
R¹ is a phenyl or pyridyl group which may be substituted at the 4-position thereof with a halogen atom selected from fluorine, chlorine or bromine or a C₁₋₆ alkoxy group;
R² is a phenyl group substituted at the 4-position thereof with a C₁₋₆ alkoxy group or a C₁₋₆ alkylthio group;
R³ is a hydrogen atom or a phenyl or pyridyl group which may be substituted by halogen atom or atoms; and
A is a C₁₋₃ alkylene group or C₃₋₄ alkenylene group.

11. The preventive and therapeutic agent of claim 9, wherein in the formula (I),
R¹ is a phenyl or pyridyl group which may be substituted at the 4-position thereof with a chlorine atom or a methoxy group;
R² is a phenyl group substituted at the 4-position thereof with a methoxy group or a methylthio group;
R³ is a hydrogen atom, phenyl group, 4-chlorophenyl group, 2-pyridyl group or 3-pyridyl group; and
A is a methylene group, ethylene group or 2-propenylene group.

12. The preventive and therapeutic agent of claim 9, wherein said active ingredient is
5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

13. Use of a pyridazine derivative represented by the following formula (I) or a derivative thereof for the production of an OPN production inhibitor: 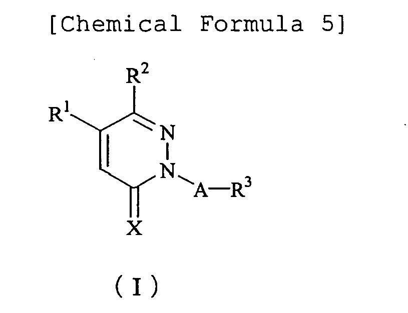 wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

14. Use of claim 13, wherein in the formula (I),
R¹ is a phenyl or pyridyl group which may be substituted at the 4-position thereof with a halogen atom selected from fluorine, chlorine or bromine or a C₁₋₆ alkoxy group;
R² is a phenyl group substituted at the 4-position thereof with a C₁₋₆ alkoxy group or a C₁₋₆ alkylthio group;
R³ is a hydrogen atom or a phenyl or pyridyl group which may be substituted by halogen atom or atoms; and
A is a C₁₋₃ alkylene group or C₃₋₄ alkenylene group.

15. Use of claim 13, wherein in the formula (I),
R¹ is a phenyl or pyridyl group which may be substituted at the 4-position thereof with a chlorine atom or a methoxy group;
R² is a phenyl group substituted at the 4-position thereof with a methoxy group or a methylthio group;
R³ is a hydrogen atom, phenyl group, 4-chlorophenyl group, 2-pyridyl group or 3-pyridyl group; and
A is a methylene group, ethylene group or 2-propenylene group.

16. Use of claim 13, wherein said active ingredient is
5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

17. Use of a pyridazine derivative represented by the following formula (I) or a derivative thereof for the production of a preventive and therapeutic agent for a disease resulting from enhanced OPN production: 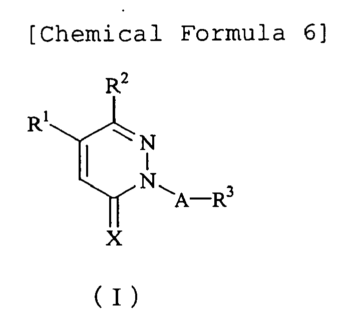 wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

18. Use of claim 17, wherein in the formula ( I),
R¹ is a phenyl or pyridyl group which may be substituted at the 4-position thereof with a halogen atom selected from fluorine, chlorine or bromine or a C₁₋₆ alkoxy group;
R² is a phenyl group substituted at the 4-position thereof with a C₁₋₆ alkoxy group or a C₁₋₆ alkylthio group;
R³ is a hydrogen atom or a phenyl or pyridyl group which may be substituted by halogen atom or atoms; and
A is a C₁₋₃ alkylene group or C₃₋₄ alkenylene group.

19. Use of claim 17, wherein in the formula (I),
R¹ is a phenyl or pyridyl group which may be substituted at the 4-position thereof with a chlorine atom or a methoxy group;
R² is a phenyl group substituted at the 4-position thereof with a methoxy group or a methylthio group;
R³ is a hydrogen atom, phenyl group, 4-chlorophenyl group, 2-pyridyl group or 3-pyridyl group; and
A is a methylene group, ethylene group or 2-propenylene group.

20. Use of claim 17, wherein the active ingredient is
5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

21. An OPN production inhibitor composition comprising a pyridazine derivative represented by the following formula (I) or a derivative thereof and a pharmaceutically acceptable carrier:
[Chemical Formula 7] 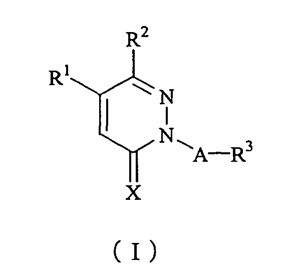 wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

22. The composition of claim 21, wherein in the formula (I),
R¹ is a phenyl or pyridyl group which may be substituted at the 4-position thereof with a halogen atom selected from fluorine, chlorine or bromine or a C₁₋₆ alkoxy group;
R² is a phenyl group substituted at the 4-position thereof with a C₁₋₆ alkoxy group or a C₁₋₆ alkylthio group;
R³ is a hydrogen atom or a phenyl or pyridyl group which may be substituted by halogen atom or atoms; and
A is a C₁₋₃ alkylene group or C₃₋₄ alkenylene group.

23. The composition of claim 21, wherein in the formula (I),
R¹ is a phenyl or pyridyl group which may be substituted at the 4-position thereof with a chlorine atom or a methoxy group;
R² is a phenyl group substituted at the 4-position thereof with a methoxy group or a methylthio group;
R³ is a hydrogen atom, phenyl group, 4-chlorophenyl group, 2-pyridyl group or 3-pyridyl group; and
A is a methylene group, ethylene group or 2-propenylene group.

24. The composition of claim 21, wherein the active ingredient is
5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

25. A preventive and therapeutic agent composition for a disease resulting from enhanced OPN production, comprising a pyridazine derivative represented by the following formula (I) or a derivative thereof and a pharmaceutically acceptable carrier: 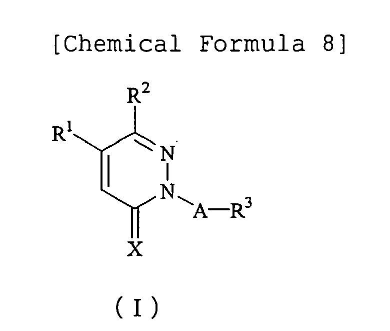 wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

26. The composition of claim 25, wherein in the formula (I).
R¹ is a phenyl or pyridyl group which may be substituted at the 4-position thereof with a halogen atom selected from fluorine, chlorine or bromine or a C₁₋₆ alkoxy group;
R² is a phenyl group substituted at the 4-position thereof with a C₁₋₆ alkoxy group or a C₁₋₆ alkylthio group;
R³ is a hydrogen atom or a phenyl or pyridyl group which may be substituted by halogen atom or atoms; and
A is a C₁₋₃ alkylene group or C₃₋₄ alkenylene group.

27. The composition of claim 25, wherein in the formula (I),
R¹ is a phenyl or pyridyl group which may be substituted at the 4-position thereof with a chlorine atom or a methoxy group;
R² is a phenyl group substituted at the 4-position thereof with a methoxy group or a methylthio group;
R³ is a hydrogen atom, phenyl group, 4-chlorophenyl group, 2-pyridyl group or 3-pyridyl group; and
A is a methylene group, ethylene group or 2-propenylene group.

28. The composition of claim 25, wherein the active ingredient is
5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

29. A therapeutic method of a disease resulting from enhanced OPN production, comprising administering an effective amount of a pyridazine derivative represented by the following formula (I) or a derivative thereof: 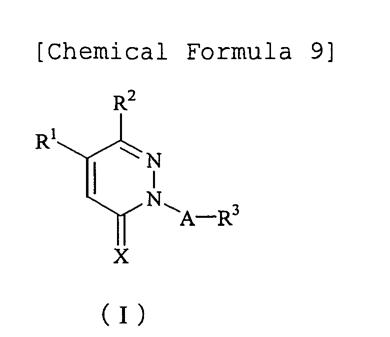 wherein:
R¹ means a phenyl or pyridyl group which may be substituted by 1 to 3 substituents selected from halogen atoms and C₁₋₆ alkoxy groups;
R² means a phenyl group which may be substituted at the 4-position thereof with a C₁₋₆ alkoxy group or C₁₋₆ alkoxythio group and may also be substituted at one or two other positions thereof a like number of substituents selected from halogen atoms, C₁₋₆ alkoxy groups and C₁₋₆ alkoxythio groups;
R³ means a hydrogen atom; a C₁₋₆ alkoxy group; a halogenated C₁₋₆ alkyl group; a C₃₋₆ cycloalkyl group; a phenyl, pyridyl or phenyloxy group which may be substituted by 1 to 3 substituents selected from halogen atoms, C₁₋₆ alkyl groups, C₁₋₆ alkoxy groups, carboxyl groups, C₂₋₇ alkoxycarbonyl groups, nitro groups, amino groups, C₁₋₆ alkylamino groups and C₁₋₆ alkylthio groups; a substituted or unsubstituted piperidino, piperidyl, piperazino or morpholino group; a substituted or unsubstituted aminocarbonyl group; a C₂₋₇ alkylcarbonyl groups; or a substituted or unsubstituted piperazinocarbonyl group;
A means a single bond, a C₁₋₆ linear or branched alkylene group, or a C₂₋₉ linear or branched alkenylene group; and
X means an oxygen atom or a sulfur atom, with a proviso that A is a single bond when R³ is a halogenated C₁₋₆ alkyl group.

30. The method of claim 29, wherein in the formula (I),
R¹ is a phenyl or pyridyl group which may be substituted at the 4-position thereof with a halogen atom selected from fluorine, chlorine or bromine or a C₁₋₆ alkoxy group;
R² is a phenyl group substituted at the 4-position thereof with a C₁₋₆ alkoxy group or a C₁₋₆ alkylthio group;
R³ is a hydrogen atom or a phenyl or pyridyl group which may be substituted by halogen atom or atoms; and
A is a C₁₋₃ alkylene group or C₃₋₄ alkenylene group.

31. The method of claim 29, wherein in the formula (I),
R¹ is a phenyl or pyridyl group which may be substituted at the 4-position thereof with a chlorine atom or a methoxy group;
R² is a phenyl group substituted at the 4-position thereof with a methoxy group or a methylthio group;
R³ is a hydrogen atom, phenyl group, 4-chlorophenyl group, 2-pyridyl group or 3-pyridyl group; and
A is a methylene group, ethylene group or 2-propenylene group.

32. The method of claim 29, wherein the active ingredient is
5-(4-chlorophenyl)-6-[4-(methylthio)phenyl]-2-(2-py ridylmethyl)-2H-pyridazine-3-thione, 5-(4-chlorophenyl)-6-[4-(methylthio)phenyl]-2-(3-py ridylmethyl)-2H-pyridazin-3-one, 5,6-bis(4-methoxyphenyl)-2-(4-chlorocinnamyl)-2H-py ridazin-3-one, 2-benzyl-5-(4-chlorophenyl)-6-[4-(methylthio)phenyl ]-2H-pyridazin-3-one, 2-(4-chlorobenzyl)-6-(4-methoxyphenyl)-5-(4-pyridin yl)-2H-pyridazin-3-one, 5,6-bis(4-methoxyphenyl)-2-ethyl-2H-pyridazin-3-one, or a salt thereof.

33. The method of claim 29, wherein said disease resulting from said enhanced OPNproduction is post-PTCA restenosis, a kidney disease, tuberculosis, sarcoidosis, cirrhosis, colorectal cancer, ovarian cancer, prostatic cancer, breast cancer, urinary calculus or myelomatous tumor.

34. The method of claim 29, wherein said disease resulting from said enhanced OPN production is multiple myeloma.


SEQUENCE LISTING

<110> Kowa Company, Ltd.

<120> Inhibiting method for Osteopontin production

<130> KW0125

<160> 4

<170> PatentIn version 3.1

<210> 1
<211> 20
<212> DNA
<213> Artificial Sequence

<220>
<223> Primer for human Osteopontin gene

<400> 1
ggactccatt gactcgaacg 20

<210> 2
<211> 20
<212> DNA
<213> Artificial Sequence

<220>
<223> Primer for human Osteopontin gene

<400> 2
taatctggac tgcttgtggc 20

<210> 3

<211>  22
<212>  DNA
<213>  Artificial Sequence

<220>
<223>  Primer for GAPDH gene

<400>  3
aattaccaca acccctacaa ac                                                22

<210>  4
<211>  21
<212>  DNA
<213>  Artificial Sequence

<220>
<223>  Primer for GAPDH gene

<400>  4
caactctgca acatcttcct c                                                 21